# EUROPEAN PATENT SPECIFICATION

(11) **EP 3 097 205 B1**
(45) Date of publication and mention of the grant of the patent: **06.03.2019**
(21) Application number: 15700097.7
(22) Date of filing: 07.01.2015
(51) Int. Cl.: C12Q 1/686, C12Q 1/6816

(54) **NUCLEIC ACID AMPLIFICATION METHOD**
VERFAHREN ZUR AMPLIFIKATION VON NUKLEINSÄURE
PROCÉDÉ D'AMPLIFICATION D'ACIDE NUCLÉIQUE

(30) Priority: 23.01.2014 EP 14152306
(43) Date of publication of application: 30.11.2016
(73) Proprietor: Qiagen GmbH, 40724 Hilden (DE)
(72) Inventor: MÜLLER, Daniel, 40724 Hilden (DE); SCHERER, Mario, 42697 Solingen (DE); QUINTEL, Harald, CH-8266 Steckborn (CH)
(74) Representative: CH Kilger Anwaltspartnerschaft mbB
(86) International application number: PCT/EP2015/050131
(87) International publication number: WO 2015/110281

(56) References cited:
- EP-A1- 2 192 198
- WO-A1-2012/083235
- TSUYOSHI ISHII ET AL: "Practical evaluation of universal conditions for four-plex quantitative PCR", ANALYTICAL AND BIOANALYTICAL CHEMISTRY, SPRINGER, BERLIN, DE, vol. 388, no. 1, 14 March 2007 (2007-03-14), pages 271-278, XP019512308, ISSN: 1618-2650, DOI: 10.1007/S00216-007-1213-3
- FAULDS KAREN ET AL: "Multiplexed detection of six labelled oligonucleotides using surface enhanced resonance Raman scattering (SERRS)", THE ANALYST, R S C PUBLICATIONS, GB, vol. 133, no. 11, 1 November 2008 (2008-11-01), pages 1505-1512, XP008100552, ISSN: 0003-2654, DOI: 10.1039/B800506K
- FEUCHTENBERGER M ET AL: "Semiquantitative and qualitative assessment of B-lymphocyte VH repertoire by a fluorescent multiplex PCR", JOURNAL OF IMMUNOLOGICAL METHODS, ELSEVIER SCIENCE PUBLISHERS B.V.,AMSTERDAM, NL, vol. 276, no. 1-2, 1 May 2003 (2003-05-01) , pages 121-127, XP004422646, ISSN: 0022-1759, DOI: 10.1016/S0022-1759(03)00101-7
- LI LI ET AL: "[Developing of a new multicolor-fluorescent labeled STR amplification kit]", MEDLINE, 8 May 2009 (2009-05-08), XP002527494,
- "AlmaKnowledgeServer Uncovers Hidden Knowledge from Medline I N T H I S I S S U E 2 NEW: Specifi c, Flexible Labeling of Proteins 4 Detect Protein Phosphorylation Directly Inside the Cell 5 NEW: Highly Specifi c Kinase Activity Assays 6 Application Note: Quantitation of Activated Transcription Factor", , 1 May 2006 (2006-05-01), XP055123699, Retrieved from the Internet: URL:http://www.activemotif.com/documents/8 9.pdf [retrieved on 2014-06-17]
- , 13 February 2009 (2009-02-13), XP055123703, Retrieved from the Internet: URL:http://www.vincibiochem.com/PDFfiles/1 634.pdf [retrieved on 2014-06-17]

## Description

### Field of the invention

The present invention is in the field of molecular biology. Particularly, the present invention relates to the detection of nucleic acids, e.g. in amplification reactions such as polymerase chain reactions (PCR). The present invention is particularly useful in genotyping, quantitative PCR, real-time PCR and multiplex-PCR. The present invention particularly relates to dyes used in PCR.

### Background

Fluorescent dyes have a wide application in analytical chemistry, biochemistry and molecular biology, such as in DNA sequencing and during detection and amplification of nucleic acids. Fluorescent dyes absorb light of a specific wavelength ("excitation") and re-emit energy at a different specific wavelength ("emission"). The fluorescent dyes are frequently used as molecular labels attached to probes or other biomolecules.

Multiplex polymerase chain reactions (multiplex PCR) is a PCR technique that enables amplification of two or more products in parallel in a single reaction tube. It is widely used in genotyping applications and different areas of molecular biology, e.g. in research, forensic and diagnostic applications, including human identification and paternity testing and for diagnosis of infectious diseases or chimerism analysis after allogeneic bone marrow transplantation. Multiplex PCR can also be used for qualitative and semi-quantitative gene expression analysis using cDNA as a starting template or in combination with reverse transcription with mRNA as starting material. The nucleic acids analyzed may for example originate from a variety of eukaryotic (human, animal or plant) and prokaryotic (bacterial) or viral sources.

Multiplex PCR is for example used for the simultaneous detection of multiple marker genes and/or their polymorphisms, e.g. short tandem repeats (STRs) or deletion insertion polymorphisms (DIPS or Indels). Detection of the amplification products and their genotyping is usually carried out by multiple color fluorescence detection after electrophorectic separation (e.g. capillary gel electrophoresis) in DNA sequencers. In real-time, quantitative multiplex PCR, the amplification of multiple target sequences can be monitored at the same time by simultaneous detection of fluorescence of different fluorescent dyes.

For the purpose of multiple colour fluorescence detection, at least one primer of each primer pair used during amplification is labeled by covalently bond fluorescent dye at is 5'-OH end or at an internal base.

To date, the simultaneous amplification of 8 to 16 or even more target sequences is possible.

For example, European patent application EP 2 192 198 discloses the use of combinations of dyes in DNA sequence analyzers in a capillary electrophoresis devices combined with a special multi-wavelength fluorescence detection system.

Ishii et al. (Anal. Bioanal. Chem. 2007, 388:271-278) have determined optimal conditions for four-color multiplex qPCR in terms of lower concentrations of probe/primers, reaction reagent, and a high-performance fluorescence-based detection instrument.

Faulds et al. (Analyst 2008, 133:1505-1512) reveal a method to evaluate the presence of a particular labelled oligonucleotide in a mixture by using partial least squares (PLS) regression in combination with surface enhanced resonance Raman scattering (SERRS). The method disclosed in Faulds *et al.* appears to be ideal for the detection of up to six labelled oligonucleotides in a mixture.

Feuchtenberger et al. (Journal of Immunological Methods 2003, 276:121-127) disclose the use of different labels in a fluorescence multiplex PCR method for VH gene family usage screening during IgH rearrangements in human B-lymphocytes.

A further PCR-based STR system for genotyping of 18 loci has been disclosed by Li et al. (Fa Yi Xue Za Zhi. 2006, 22(2):111-116) by using primers labeled with four fluorescent dyes.

However, modern thermocyclers (for real-time PCR) and DNA sequencers used in multiplex PCR are adapted for the simultaneous detection of 3 to 5 different fluorescent dyes. Usually one of the fluorescent dyes is used as a label of a length standard when gel electrophoresis analysis is performed.

The apparatuses used for detection are often designed and calibrated for the use of combinations of very specific fluorescent dyes, i.e. in terms of excitation and detection wavelengths and filters used. The optical set-up of many devices encompasses the use of one Argon laser (488nm) or a solid state diode (505 nm), a spectrograph, virtual filters, in combination with a CCD camera and algorithms for spectral calculation. This set-up severely limits the amount of dyes that may be used.

At the same time the identification of a dye in the spectral emission region 605 to 630 nm is difficult. These are difficult to excite by the known and used lasers. However the sensitivity in this range is the relevant factor for the overall sensitivity of the set-up. Hence, it would be desirable to have an ideal dye also in this spectral range.

### Brief description of the invention

The present invention relates to specific combinations of fluorescent dyes for the detection of fluorescently labeled nucleic acids.

The invention relates to a method for the simultaneous detection of at least six nucleic acids labeled with a covalently attached dye in a sample comprising the step of:
detecting the fluorescence emission of said fluorescent dyes upon excitation, wherein the at least six dyes attached to said nucleic acids are selected from the groups of:
i. 6-FAM or 5-FAM or a blend thereof covalently attached to a first nucleic acid,
ii. DY-530, HEX or ATTO 532 or a blend thereof covalently attached to a second nucleic acid,
iii. ATTO550 or DY-555 or a blend thereof covalently attached to a third nucleic acid,
iv. ATTO565, DY510-XL or ROX or a blend thereof covalently attached to a fourth nucleic acid,
v. DY632 or DY520-XL or a blend thereof covalently attached covalently attached to a fifth nucleic acid and,
vi. Chromeo 494 attached to a sixth nucleic acid.

The invention also relates to a method for the simultaneous detection of at least six amplification products from an amplification reaction, comprising the steps of
(i) amplifying five or more loci on nucleic acid templates using primers or pairs of primers substantially complementary to sequences flanking said loci; and
(ii) detecting said amplification products by fluorescence detection of fluorescently labelled amplification products or alternatively by fluorescence detection of fluorescently labelled oligonucleotide probes complementary to sequences on said loci of said amplification products,
(iii) wherein a fluorescent dye is covalently attached to said amplification product and/or to said oligonucleotide probe and/or to said primer and/or to at least one primer of said primer pair and optionally to a size marker,
(iv) and wherein at least six different dyes are used for differentiating the at least six amplification products wherein, the dyes may be selected from the group comprising:
   i. a first dye which is 6-FAM or 5-FAM or a blend thereof,
   ii. a second dye which is DY-530, HEX or ATTO 532 or a blend thereof,
   iii. a third dye which is ATTO550 or DY-555 or a blend thereof,
   iv. a fourth dye which is ATTO565, DY-510XL or ROX or a blend thereof,
   v. a fifth dye which is DY632 or DY520-XL or a blend thereof,
   vi. and a sixth dye which is Chromeo 494 and,
   the invention relates to a kit for multiplex PCR or PCR comprising at least six different fluorescent dyes suitable for the covalent attachment to nucleotides, primers, size markers or oligonucleotide probes used in multiplex PCR, wherein the six dyes are selected from the six groups below:
   i. 6-FAM or 5-FAM or a blend thereof,
   ii. DY-530, HEX or ATTO 532 or a blend thereof,
   iii. ATTO550 or DY-555 or a blend thereof,
   iv. ATTO565, DY-510XL or ROX or a blend thereof covalently, or
   v. DY632, and
   vi. Chromeo 494,

### Detailed description of the invention

The dye collection described herein for the first time provides for higher sensitivity, less cross talk between color channels and compatibility with numerous existing devices.

The present invention relates to specific combinations of fluorescent dyes for the detection of fluorescently labeled nucleic acids. The structures of 5-FAM (5-carboxyfluorescin) and 6-FAM (6-carboxyfluorescin) are illustrated in the figures. The structures of HEX (6-carboxy-2',4,4',5',7,7'-hexachlorofluorescein) and ROX (5-carboxy-X-rhodamine) are illustrated in the figures. The structures of DY-530, DY-555, DY-556, DY-510XL, DY-632 and DY-520XL are illustrated in the figures. The structures of ATTO 532, ATTO 550 and ATTO 565 are illustrated in the figures. DY-530, DY-555, DY-556, DY-510XL, DY-632 and DY-520XL are products of Dyomics GmbH, Jena, Germany. ATTO 532, ATTO 550 and ATTO 565 are products of ATTO-TEC GMBH, Siegen, Germany. Chromeo™ 494 is a product of Active Motif, Carlsbad, California, US.

In a preferred embodiment, the invention relates to a method for the simultaneous detection of at least six nucleic acids labeled with a covalently attached dye in a sample comprising the step of:
detecting the fluorescence emission of said fluorescent dyes upon excitation, wherein the at least six dyes attached to said nucleic acids are selected from the groups of:
i. 6-FAM or 5-FAM or a blend thereof covalently attached to a nucleic acid,
ii. DY-530, HEX or ATTO 532 or a blend thereof covalently attached to a nucleic acid,
iii. ATTO550 or DY-555 or a blend thereof covalently attached to a nucleic acid,
iv. ATTO565, DY510-XL or ROX or a blend thereof covalently attached to a nucleic acid,
v. DY632 or DY520-XL or a blend thereof covalently attached to a nucleic acid and,
vi. Chromeo 494 attached to a nucleic acid.

In a preferred embodiment, the invention relates to a method for the simultaneous detection of at least six amplification products from an amplification reaction, comprising the steps of:
(i) amplifying five or more loci on nucleic acid templates using primers or pairs of primers substantially complementary to sequences flanking said loci; and
(ii) detecting said amplification products by fluorescence detection of fluorescently labelled amplification products or alternatively by fluorescence detection of fluorescently labelled oligonucleotide probes complementary to sequences on said loci of said amplification products,
(iii) wherein a fluorescent dye is covalently attached to said amplification product and/or to said oligonucleotide probe and/or to said primer and/or to at least one primer of said primer pair and optionally to a size marker,
(iv) and wherein at least six different dyes are used for differentiating the at least six amplification products wherein, the dyes may be selected from the group comprising:
   i. a first dye which is 6-FAM or 5-FAM or a blend thereof,
   ii. a second dye which is DY-530, HEX or ATTO 532 or a blend thereof,
   iii. a third dye which is ATTO550 or DY-555 or a blend thereof,
   iv. a fourth dye which is ATTO565, DY510-XL or ROX or a blend thereof,
   v. a fifth dye which is DY632 or DY520-XL or a blend thereof,
   vi. and a sixth dye which is Chromeo 494.

As outlined above, the invention relates to combinations of at least six different fluorescent dyes. In the context of the above described methods this means that when a size marker fluorescently labelled with one dye of the invention is used, at least five nucleic acid sequences (i.e. loci) can be amplified and detected, depending on whether a combination of four, five or even more different dyes is used. If no fluorescently labelled size marker is used, then at least four, five, or six sequences can be amplified and detected.

A "locus" in the context of the present invention is a part of the sequence of a nucleic acid, e.g. a chromosome, mRNA, plasmid, cosmid, DNA or RNA (of any origin, e.g. bacterial, viral, eukaryotic, prokaryotic, from plants, fungi or animals, e.g. of human origin) and the like, that is to be amplified and/or detected.

"Amplification products" herein are nucleic acids or oligonucleotides that are the product of an amplification reaction, e.g. of a polymerase chain reaction. They are for example defined by the primers used for amplification.

A "primer" herein refers to an oligonucleotide comprising a sequence that is complementary to a nucleic acid to be amplified or transcribed ("template"). During replication polymerases attach nucleotides to the 3'-OH end of the primer complementary to the respective nucleotides of the template.

An "oligonucleotide" herein refers to a stretch of nucleic acid, e.g. RNA or DNA, that comprises a sequence of two or more nucleotides, e.g. between 2 and 250 nucleotides, more preferably between 2 and 200, even more preferably between 2 and 100, even more preferably between 2 and 30, even more preferably between 2 and 25, even more preferably between 2 and 20, even more preferably between 5 and 25, and most preferably between 10 and 25 nucleotides.

In the first aspect of the method, during amplification detection may for example occur using fluorescently labelled oligonucleotide probes. Alternatively fluorescently labelled primers may be used. In another alternative, fluorescently labelled nucleotides may be used that are incorporated into the amplification products. In this case it is preferred that for every locus to be amplified or detected, the amplification is performed in a separate reaction tube. The amplification products may then be unified for detection. Thus, detection may in some cases occur during amplification, e.g. after or during each cycle of a polymerase chain reaction and/or after the complete amplification reaction.

In one embodiment of the first aspect of the method, the method additionally comprises the step of separating the amplification products by their size or molecular weight before detecting said amplification products. Separation by size or molecular weight can e.g. be performed using electrophoresis, e.g. gel electrophoresis, or chromatographical techniques.

The amplification reaction is preferably selected from the group comprising polymerase chain reaction (PCR), ligase chain reaction (LCR), transcription-based amplification system (TAS), nucleic acid sequence based amplification (NASBA), rolling circle amplification (RCA), transcription-mediated amplification (TMA), self-sustaining sequence replication (3SR), QP amplification and (thermostable) helicase dependent amplification ((t)HAD). More preferably the amplification reaction is a PCR. Most preferably, the amplification reaction is a multiplex PCR, i.e. the amplification of more than one target nucleic acid sequence in a single tube, e.g. 2, 3, 4, 5, 6, 7, 8, 9, 10, 11, 12, 13, 14, 15,16, 17,18, 19, 20, 21, 22, 23, 24, 25, 26, 27, 28, 29, 30, 31, 32, 33, 34, 35, 36, 37, 38, 39 or 40 loci (target nucleic acid sequences) may be amplified simultaneously.

As outlined above, the nucleic acid sequences (i.e. the loci) may be amplified simultaneously, e.g. in one reaction tube or separately, e.g. in different reaction tubes. It is preferred in the methods of the present invention that said loci are amplified simultaneously.

Preferably, the amplification reaction is a multiplex PCR.

Preferably, the reaction additionally comprises the step of separating the amplification products by their size or molecular weight before detecting said amplification products.

Preferably the amplification reaction is real-time multiplex polymerase chain reaction and the method comprises the steps of:
(i) simultaneously amplifying at least six loci on nucleic acid templates using pairs of primers substantially complementary to sequences flanking said loci;
(ii) detecting said amplification products using at least one oligonucleotide probe for each locus to be amplified, wherein said oligonucleotide probe is substantially complementary to a sequence on said locus to be amplified,
(iii) wherein a fluorescent dye is covalently attached to each oligonucleotide probe and wherein at least six different dyes are used for differentiating the at least six probes wherein, the dyes may be selected from the group comprising:
   i. a first dye which is 6-FAM or 5-FAM or a blend thereof,
   ii. a second dye which is DY-530, HEX or ATTO 532 or a blend thereof,
   iii. a third dye which is ATTO550 or DY-555 or a blend thereof,
   iv. a fourth dye which is ATTO565, DY510-XL or ROX or a blend thereof,
   v. a fifth dye which is DY632 or DY520-XL or a blend thereof,
   vi. and a sixth dye which is Chromeo 494.

Here, one combination is preferred, i.e. 6-FAM, DY-530, ATTO565, ATTO550, DY510-XL, DY632 and Chromeo 494.

Ideally, the first dye is 6-FAM, the second dye is ATTO550 the third dye is ATTO565, and the fourth dye is Chromeo 494.

If more than four dyes are used and these are preferably 6-FAM, DY-530, ATTO550, ATTO565, DY510-XL, DY632 and Chromeo 494.

If six dyes are used these are preferably 6-FAM, DY-530, ATTO550, ATTO565, DY632 and Chromeo 494.

If more than six dyes are used these are preferably selected from the group comprising 6-FAM, DY-530, ATTO550, ATTO565, DY510-XL, DY632 and Chromeo 494.

If more than six dyes are used these are preferably selected from the group consisting of 6-FAM, DY-530, ATTO550, ATTO565, DY510-XL, DY632 and Chromeo 494.

Preferably, at least one dye is covalently attached to an oligonucleotide size marker.

The invention also relates to a composition comprising:
(i) a first nucleic acid to which a fluorescent dye selected from the group consisting of 6-FAM and 5-FAM or a blend thereof is covalently attached;
(ii) a second nucleic acid to which a fluorescent dye selected from the group consisting of DY-530, HEX or ATTO 532 or a blend thereof is covalently attached;
(iii) a third nucleic acid to which a fluorescent dye selected from the group consisting of ATTO550 and DY-555 is covalently attached;
(iv) a fourth nucleic acid to which a fluorescent dye selected from the group consisting of ATTO565, DY510-XL and ROX is covalently attached;
(v) a fifth nucleic acid to which a fluorescent dye selected from the group consisting of DY632 or DY520-XL or a blend thereof is covalently attached, and
(vi) a sixth nucleic acid to which a fluorescent dye is attached, the dye being Chromeo 494.

Such a composition may be a pre-mix for a PCR reaction or a multiplex PCR reaction. It may comprise other components as a buffer or an enzyme.

In a further embodiment the invention relates to a kit for multiplex PCR or PCR comprising at least six different fluorescent dyes suitable for the covalent attachment to nucleotides, primers, size markers or oligonucleotide probes used in multiplex PCR, wherein the six dyes are selected from the six groups below:
i. 6-FAM or 5-FAM or a blend thereof,
ii. DY-530, HEX or ATTO 532 or a blend thereof,
iii. ATTO550 or DY-555 or a blend thereof,
iv. ATTO565, DY510-XL or ROX or a blend thereof,
v. DY632 or DY520-XL or a blend thereof, and
vi. Chromeo 494,

In a further embodiment the invention relates to a kit comprising different dyes, these being:
a. 6-FAM,
b. DY-530,
c. ATTO550,
d. ATTO565
e. DY632 and,
f. Chromeo 494.

In one embodiment the amplification is an amplification of a forensic sample.

### Example

Object of the invention is the use of six defined fluorescent dyes that allow simultaneous detection of PCR products in capillary electrophoresis (CE). The challenge in choosing the fluorescent dyes is dependent on many factors. Forensic labs mainly use CE instruments from ABI (ThermoFisher). The optical setup of these devices consists of a single Argon- (488nm) or "solide state" diode-laser (505nm), a spectrograph, virtual filter sets in combination with CCD-cameras and algorithms for spectral calibration, which is entirely different from other CE-instruments or real-time PCR thermocyclers. This specific optical setup strongly limits compatibility of various dyes and thus the repertoire thereof. The aim is to achieve a minimal overlap of emission spectra of six different fluorescent dyes, a higher sensitivity, less crosstalk between the color channels, as well as compatibility with the ABI 3500 CE instrument. A panel of dyes were studied that meet these criteria while matching to the colors of a fluorescence dye combination, which can be used for DNA oligonucleotide labeling and simultaneous detection by DNA sequencing automates, consisting of 6-FAM, DY-530, ATTO550, DY510-XL and DY632. Fig. 1 shows the spectral profile of this matrix standard.

Literature, patent, and internet searches revealed a panel of dyes that, according to the manufacturer, exhibit emission peaks within the defined spectral emission range (peak emission between 592 nm and 641 nm) for the sixth dye (Table 1).

| **Name of Fluorescence Dye** | **Abs Max [nm]** | **Em Max [nm]** | **Source** | **Matrix Setup** | **Baseline** | **Sensitivity** | **Comment** |
|---|---|---|---|---|---|---|---|
| ATTO 565 | 563 | 592 | ATTO-TEC | failed | na | na | |
| Dy-590 | 580 | 599 | Dyomics | failed | na | na | |
| ROX, 5-Isomer | 574 | 602 | Biomers | failed | na | na | |
| Atto Rho12 | 576 | 603 | ATTO-TEC | failed | na | na | |
| Texas Red®-X, 2/4 | 583 | 603 | Biomers | failed | na | na | |
| California Red | 583 | 603 | AAT Bioquest | successful | + | o | |
| Sunnyvale Red | 576 | 605 | AAT Bioquest | successful | o | o | |
| Tide Fluor™ 4 (TF4) | 588 | 610 | AAT Bioquest | failed | na | na | |
| Atto Rho101 | 586 | 610 | ATTO-TEC | failed | na | na | |
| CAL Fluor RED 610 | 590 | 610 | Biosearch | failed | na | na | |
| CF™594 | 593 | 614 | Biotium | failed | na | na | |
| ATTO 590 | 594 | 624 | ATTO-TEC | successful | o | reduced | reduced sensitivity |
| ATTO 594 | 601 | 627 | ATTO-TEC | successful | o | reduced | reduced sensitivity |
| Chromeo 494 | 494 | 628 | Active Motif Chromeon | successful | + | + | |
| Dy-610 | 609 | 629 | Dyomics | o | na | reduced | too high amount of PCR product required for matrix calibration --> indicates low sensitivity |
| Dy-480 XL | 500 | 630 | Dyomics | successful | + | o | |
| Eterneon | 480 | 635 | Jena Bioscience | failed | na | na | |
| ATTO 620 | 619 | 643 | ATTO-TEC | o | na | reduced | too high amount of PCR product required for matrix calibration --> indicates low sensitivity |
| DY-615 | 621 | 641 | Dyomics | o | na | reduced | too high amount of PCR product required for matrix calibration --> indicates low sensitivity |

Table 1 shows a list of candidates for the sixth dye as well as the source, feasibility of a CE-matrix with the dye, amount of background signal, and sensitivity. Six candidates could be calibrated successfully, of which five dyes yielded too high background signals or too low signal intensities. Only one dye (Chromeo 494) showed a very low background and strong signal intensity in combination with a five color dyeset, e.g. consisting of 6-FAM, DY-530, ATTO550, ATTO565 and DY632. A high sensitivity of the multiplex PCR assay is achieved by the low background noise and the strong signal intensity. This high sensitivity is a key factor, especially in forensic analyses coping with minuscule amounts of DNA samples.

The crosstalk into the other color channels is minimized by the matching spectrum of the Chromeo 494 with respect to a five color dyeset, e.g. consisting of 6-FAM, DY-530, ATTO550, ATTO565 and DY632. This reduces the occurrence of artefact peaks and facilitates the evaluation and interpretation of results.

This dye combination allows the amplification of more than 20 STR markers in a multiplex PCR and subsequent discrete separation by CE. This results in a distinct separation of single markers while retaining a reduced maximum length of PCR amplicons.

### Figure Captions

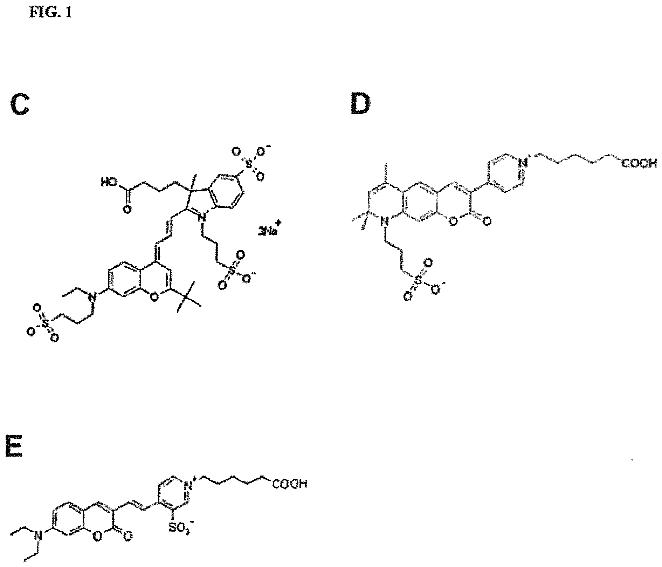

Figure 1: Structures of fluorescence dyes from Dyomics GmbH (Jena, Germany) (A) DY-530 Free carboxylic acid, (B) DY-556 NHS-ester, mono-sodium salt, (C) DY-632 free acid, di-sodium salt, (D) DY-510XL free carboxylic acid, (E) DY-520XL, carboxylic acid. In case of (A), (C), (D) and (E) free carboxyl groups are used for NHS-coupling to the 5'-ends of oligonucleotides.

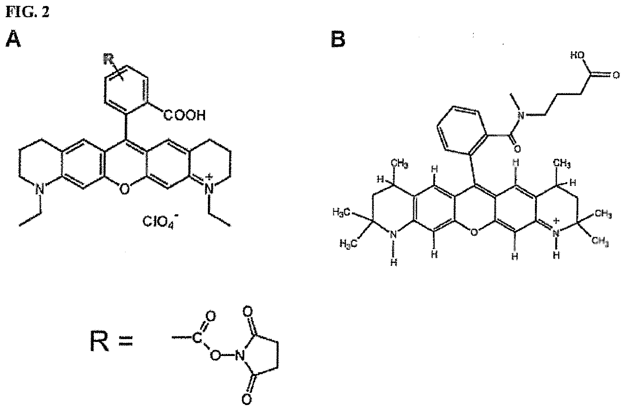

Figure 2: Structures of fluorescence dyes from ATTO-TEC GMBH (Siegen, Germany) (A) ATTO 565 (perchlorate) NHS-ester, (B) ATTO 550, free acid of one diasteriomer. The free carboxyl Group is used for NHS-coupling to the 5'-ends of oligonucleotides.

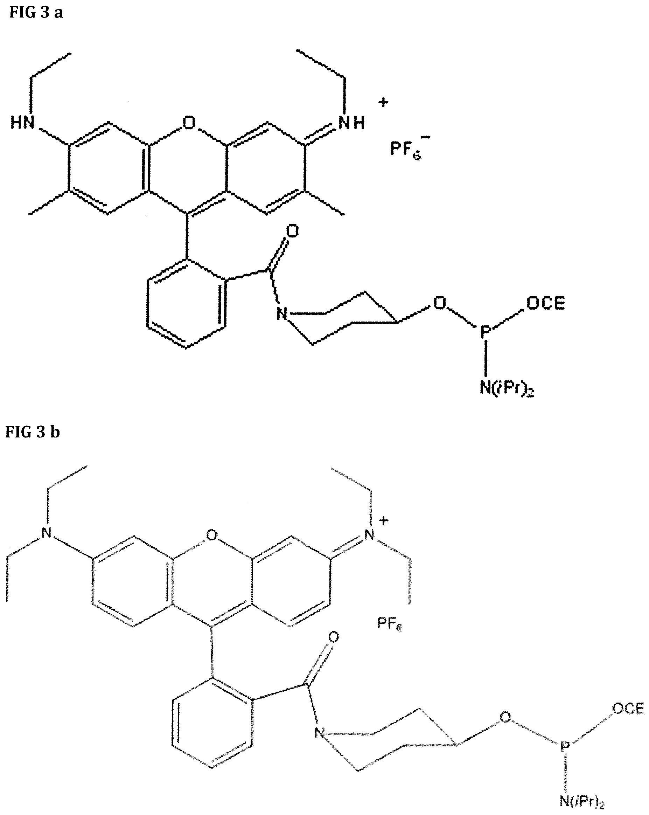

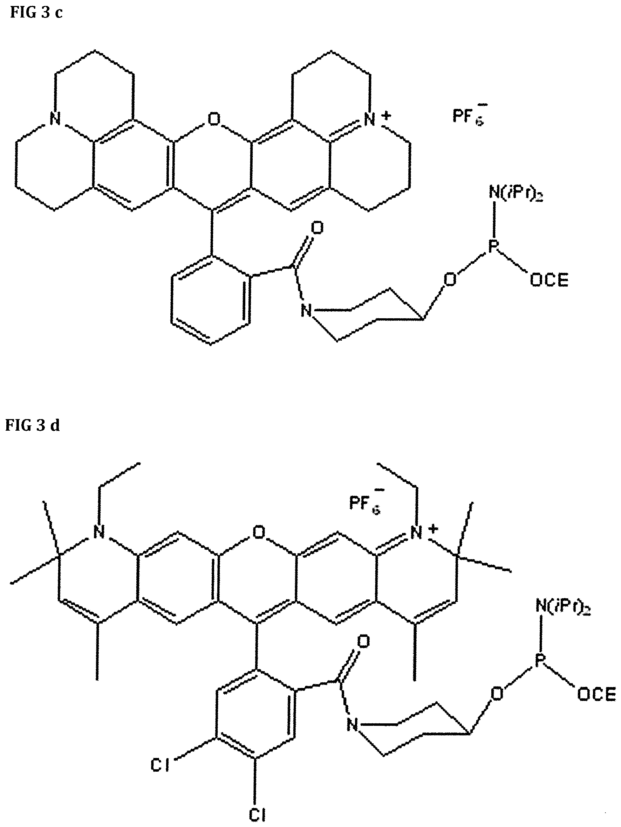

Figure 3: Structures of fluorescence dye amidires from Biosearch Technologies Inc. (Novato, CA, USA) (A) CAL Fluor Orange 560, (B) CAL Fluor Red 590, (C) CAL Fluor Red 610, (D) CAL Fluor Red 635. z'Pr: isopropyl, CE: J3-cyanoethyl.

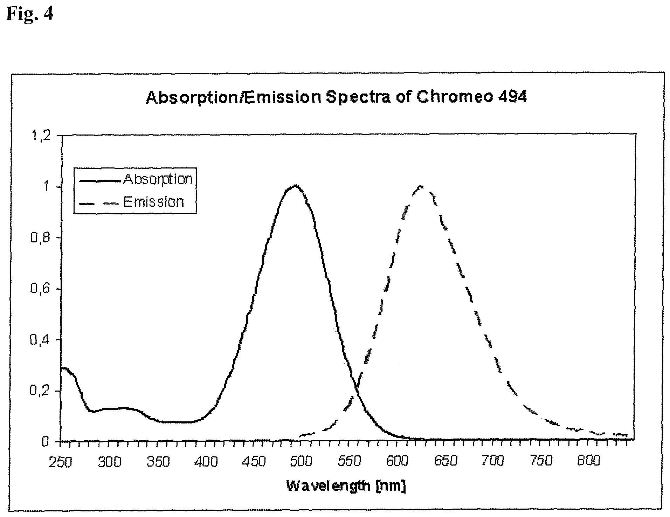

Figure 4: Shows the absorption and emission spectra of Chromeo 494.

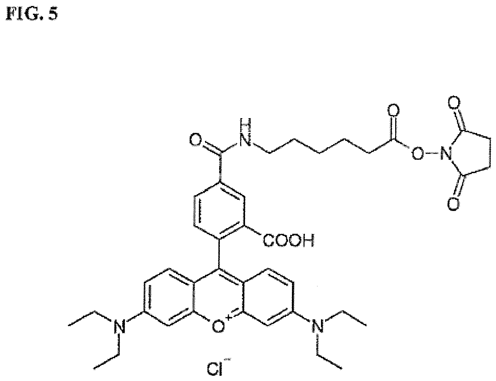

Figure 5: Structures of fluorescence dye DY-555 (Dyomics GmbH (Jena, Germany)). Structure of NHS-ester of DY-555, chloride salt.

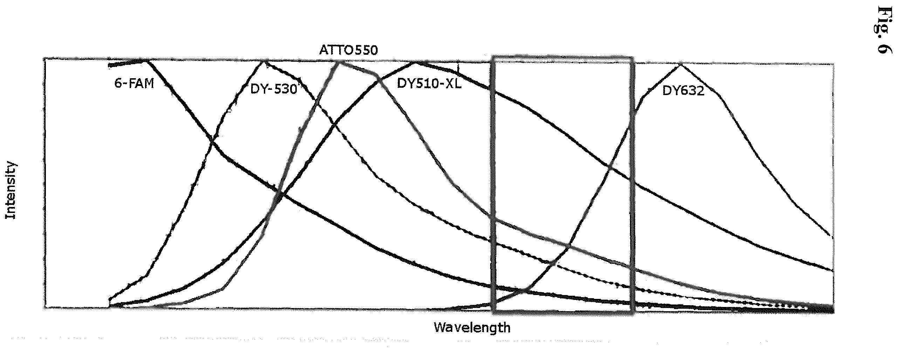

Fig. 6: Spectral profile of a five color dyeset consisting of 6-FAM, DY-530, ATTO550, DY510-XL and DY632. The box marks the free spectrum available for the sixth dye.

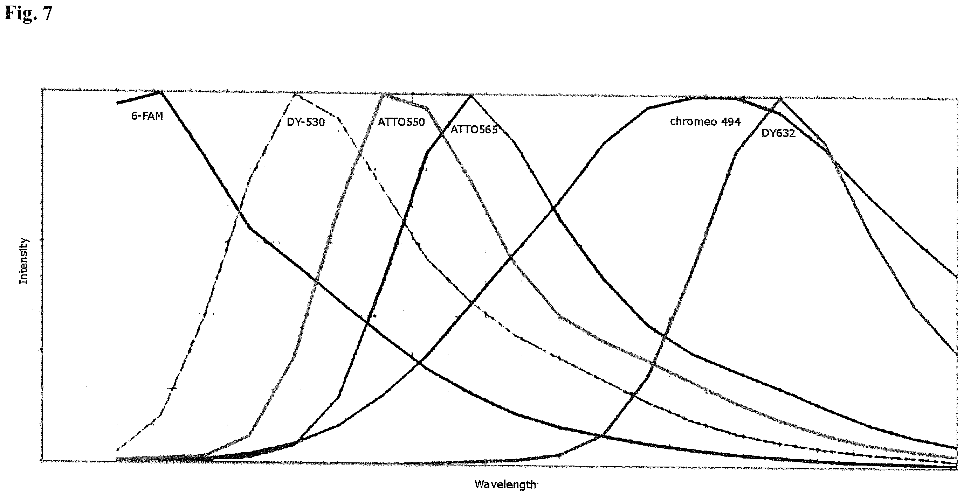

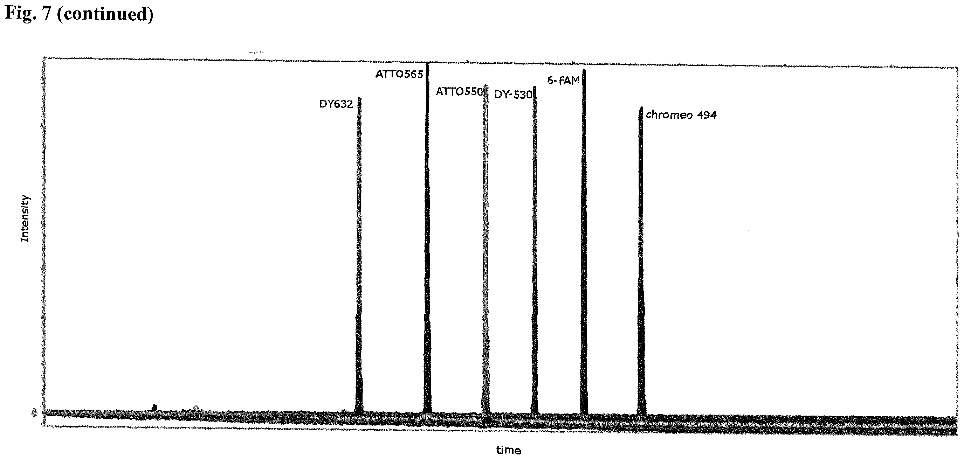

Fig. 7 shows the results of a CE matrix calibration with the dye Chromeo 494. In the upper figure spectra of six dyes including Chromeo 494 are shown. The emission peak of Chromeo 494 is positioned exactly between the peaks of the red and orange dye. This allows calibration of six dyes and the general calibration of the matrix. In the lower figure the separation of dye-labelled DNA fragments by size in bp is shown. The signal intensities of the six dyes are very similar with respect to each other. The strong signals of Chromeo 494 result in an identical sensitivity of PCR-products in the purple channel in comparison to the other channels. The background signals are very low.

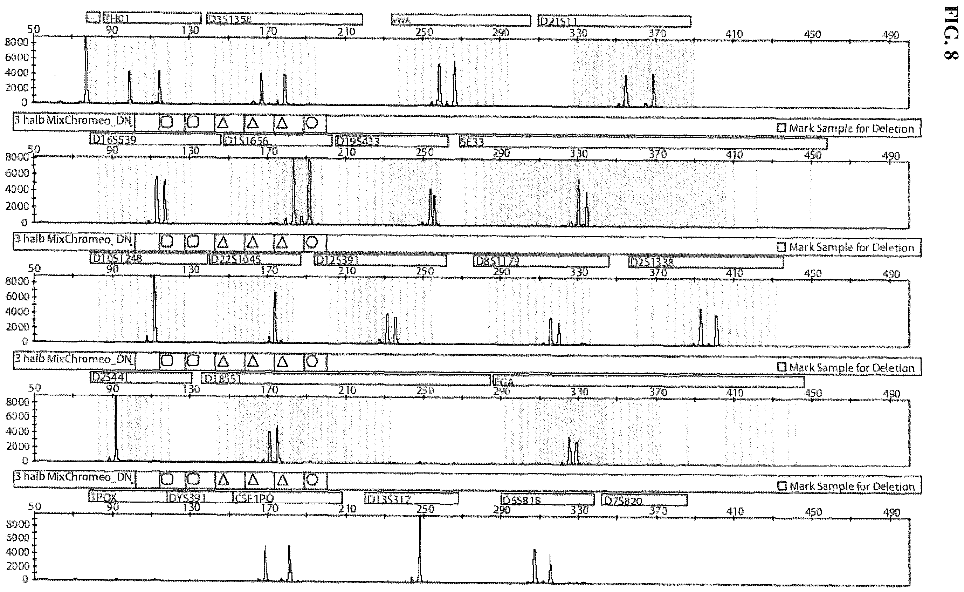

Fig. 8 shows the results of a multiplex PCR in which the forward primers of the systems CSF1PO, D13S317 and D5S818 were labelled with Chromeo 494 as the sixth dye. 500pg human DNA was used as template. The signal intensities of PCR products labelled with Chromeo 494 (purple, last panel) are sufficiently strong (approx. 8000 rfu), are comparable to those of the other channels and enable a high PCR sensitivity.

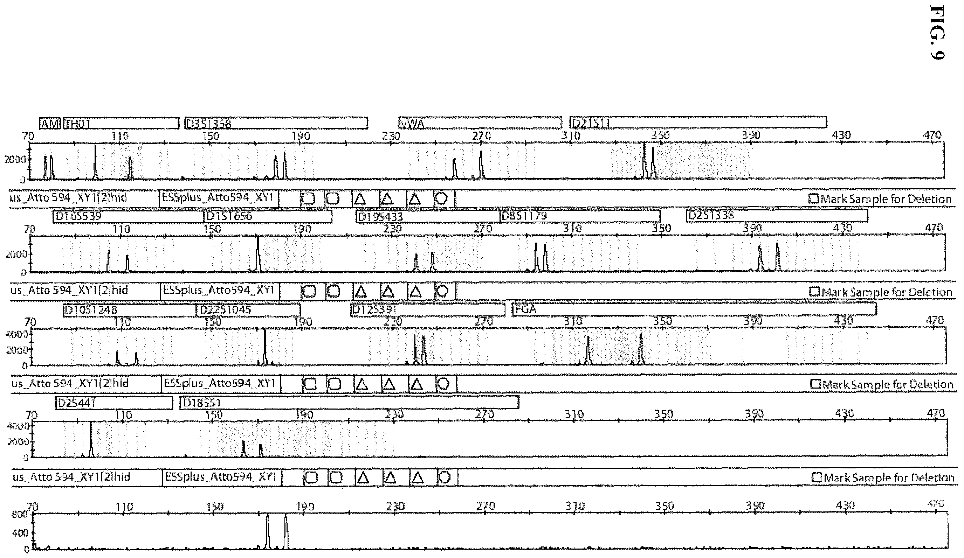

Fig. 9 shows results of a multiplex-PCR in which the forward primer of the system CSF1PO is labelled with ATTO 594 as the sixth dye. 500pg human DNA was used as template. The signal intensities of PCR-products labelled with ATTO 594 (purple, last panel) are substantially lower than those of the other channels (approx. 800 rfu to 4000 rfu.

## Claims

1. Method for the simultaneous detection of at least six nucleic acids labeled with a covalently attached dye in a sample comprising the step of:
detecting the fluorescence emission of said fluorescent dyes upon excitation, wherein the at least six dyes attached to said nucleic acids are selected from the groups of:
i. 6-FAM or 5-FAM or a blend thereof covalently attached to a nucleic acid,
ii. DY-530, HEX or ATTO 532 or a blend thereof covalently attached to a nucleic acid,
iii. ATTO550 or DY-555 or a blend thereof covalently attached to a nucleic acid,
iv. ATTO565, DY510-XL or ROX or a blend thereof covalently attached to a nucleic acid,
v. DY632 or DY520-XL or a blend thereof covalently attached to a nucleic acid and,
vi. Chromeo 494 attached to a nucleic acid.

2. Method for the simultaneous detection of at least six amplification products from an amplification reaction, comprising the steps of
(i) amplifying five or more loci on nucleic acid templates using primers or pairs of primers substantially complementary to sequences flanking said loci; and
(ii) detecting said amplification products by fluorescence detection of fluorescently labelled amplification products or alternatively by fluorescence detection of fluorescently labelled oligonucleotide probes complementary to sequences on said loci of said amplification products,
(iii) wherein a fluorescent dye is covalently attached to said amplification product and/or to said oligonucleotide probe and/or to said primer and/or to at least one primer of said primer pair and optionally to a size marker,
(iv) and wherein at least six different dyes are used for differentiating the at least six amplification products wherein, the dyes may be selected from the group comprising:
i. a first dye which is 6-FAM or 5-FAM or a blend thereof,
ii. a second dye which is DY-530, HEX or ATTO 532 or a blend thereof,
iii. a third dye which is ATTO550 or DY-555 or a blend thereof,
iv. a fourth dye which is ATTO565, DY510-XL or ROX or a blend thereof,
v. a fifth dye which is DY632 or DY520-XL or a blend thereof,
vi. and a sixth dye which is Chromeo 494.

3. Method according to claim 2, wherein the amplification reaction is selected from the group comprising polymerase chain reaction (PCR), ligase chain reaction (LCR), transcription-based amplification system (TAS), nucleic acid sequence based amplification (NASBA), rolling circle amplification (RCA), transcription-mediated amplification (TMA), self-sustaining sequence replication (3SR), Qβ amplification and (thermostable) helicase dependent amplification ((t)HAD).

4. Method according to any one of the claims 2 to 3, wherein said loci are amplified simultaneously.

5. Method according to claim 3 or 4, wherein the amplification reaction is a multiplex PCR.

6. Method according to any one of the claims 2 to 5, additionally comprising the step of separating the amplification products by their size or molecular weight before detecting said amplification products.

7. Method according to claim 2, wherein the amplification reaction is real-time multiplex polymerase chain reaction and the method comprises the steps of
(i) simultaneously amplifying at least six loci on nucleic acid templates using pairs of primers substantially complementary to sequences flanking said loci;
(ii) detecting said amplification products using at least one oligonucleotide probe for each locus to be amplified, wherein said oligonucleotide probe is substantially complementary to a sequence on said locus to be amplified,
(iii) wherein a fluorescent dye is covalently attached to each oligonucleotide probe and wherein at least six different dyes are used for differentiating the at least six probes wherein, the dyes may be selected from the group comprising:
i. a first dye which is 6-FAM or 5-FAM or a blend thereof,
ii. a second dye which is DY-530, HEX or ATTO 532 or a blend thereof,
iii. a third dye which is ATTO550 or DY-555 or a blend thereof,
iv. a fourth dye which is ATTO565, DY510-XL or ROX or a blend thereof,
v. a fifth dye which is DY632 or DY520-XL or a blend thereof,
vi. and a sixth dye which is Chromeo 494.

8. The method according to any one of the claims 1 to 7, wherein
a. the first dye is 6-FAM,
b. the second dye is ATTO550,
c. the third dye is ATTO565,
d. and the fourth dye is Chromeo 494.

9. The method according to any one of the claims 1 to 8, wherein more than six dyes are used and these are:
a. 6-FAM,
b. DY-530,
c. ATTO550,
d. ATTO565
e. DY632 and,
f. Chromeo 494.

10. The method according to any one of claims 2 to 9, wherein at least one dye is covalently attached to an oligonucleotide size marker.

11. Composition comprising:
(i) a first nucleic acid to which a fluorescent dye selected from the group consisting of 6-FAM and 5-FAM or a blend thereof is covalently attached;
(ii) a second nucleic acid to which a fluorescent dye selected from the group consisting of DY-530, HEX or ATTO 532 or a blend thereof is covalently attached;
(iii) a third nucleic to which a fluorescent dye selected from the group consisting of ATTO550 and DY-555 is covalently attached;
(iv) a fourth nucleic acid to which a fluorescent dye selected from the group consisting of ATTO565, DY510-XL and ROX is covalently attached;
(v) a fifth nucleic acid to which a fluorescent dye selected from the group consisting of DY632 or DY520-XL or a blend thereof is covalently attached, and
(vi) a sixth nucleic acid to which a fluorescent dye is attached, the dye being Chromeo 494.

12. A kit for multiplex PCR or PCR comprising at least six different fluorescent dyes suitable for the covalent attachment to nucleotides, primers, size markers or oligonucleotide probes used in multiplex PCR, wherein the six dyes are selected from the six groups below:
i. 6-FAM or 5-FAM or a blend thereof,
ii. DY-530, HEX or ATTO 532 or a blend thereof,
iii. ATTO550 or DY-555 or a blend thereof,
iv. ATTO565, DY510-XL or ROX or a blend thereof,
v. DY632 or DY520-XL or a blend thereof, and
vi. Chromeo 494.

13. The kit according to claim 12 comprising at least six different dyes these being:
a. 6-FAM,
b. DY-530,
c. ATTO550,
d. ATTO565
e. DY632 and,
f. Chromeo 494.

## Patentansprüche

1. Verfahren zum gleichzeitigen Nachweis von mindestens sechs Nukleinsäuren, die mit einem kovalent gebundenen Farbstoff in einer Probe markiert sind, umfassend den Schritt:
Nachweisen der Fluoreszenzemission der Fluoreszenzfarbstoffe bei Anregung, wobei die mindestens sechs an die Nukleinsäuren gebundenen Farbstoffe ausgewählt sind aus den Gruppen:
i. 6-FAM oder 5-FAM oder eine Mischung davon, die kovalent an eine Nukleinsäure gebunden ist,
ii. DY-530, HEX oder ATTO 532 oder eine Mischung davon, die kovalent an eine Nukleinsäure gebunden ist,
iii. ATTO550 oder DY-555 oder eine Mischung davon, die kovalent an eine Nukleinsäure gebunden ist,
iv. ATTO565, DY510-XL oder ROX oder eine Mischung davon, die kovalent an eine Nukleinsäure gebunden ist,
v. DY632 oder DY520-XL oder eine Mischung davon, die kovalent an eine Nukleinsäure gebunden ist,
vi. Chromeo 494 gebunden an eine Nukleinsäure.

2. Verfahren zum gleichzeitigen Nachweis von mindestens sechs Amplifikationsprodukte von einer Amplifikationsreaktion, umfassend den Schritten:
(i) Amplifizieren von fünf oder mehr Loci auf Nukleinsäuresonden unter Verwendung von Primern oder Primerpaaren, die im Wesentlichen komplementär zu den Sequenzen sind, die die Loci flankieren; und
(ii) Detektieren der Amplifikationsprodukte durch Fluoreszenzdetektion von fluoreszenzmarkierten Amplifikationsprodukten oder alternativ durch Fluoreszenzdetektion von fluoreszenzmarkierten Oligonukleotidsonden, die komplementär zu Sequenzen an den Loci der Amplifikationsprodukte sind,
(iii) wobei ein Fluoreszenzfarbstoff kovalent an das Amplifikationsprodukt und/oder an die Oligonukleotidsonde und/oder an den Primer und/oder an mindestens einen Primer des Primerpaares und gegebenenfalls an einen Größenmarker gebunden ist,
(iv) und wobei zur Differenzierung der mindestens sechs Amplifikationsprodukte mindestens sechs verschiedene Farbstoffe verwendet werden, wobei die Farbstoffe ausgewählt sein können aus der Gruppe, umfassend:
i. ein erster Farbstoff, der 6-FAM oder 5-FAM, oder eine Mischung davon ist,
ii. ein zweiter Farbstoff, der DY-530, HEX oder ATTO 532 oder eine Mischung davon ist,
iii. ein dritter Farbstoff, der ATTO550 oder DY-555 oder eine Mischung davon ist,
iv. ein vierter Farbstoff der ATTO565, DY510-XL oder ROX oder eine Mischung davon ist,
v. ein fünfter Farbstoff der DY632 oder DY520-XL oder eine Mischung davon ist,
vi. und ein sechster Farbstoff der Chromeo 494 ist.

3. Verfahren gemäß Anspruch 2, wobei die Amplifikationsreaktion ausgewählt ist aus der Gruppe umfassend Polymerasekettenreaktion (PCR), Ligasekettenreaktion (LCR), Transkriptions-basiertes Amplifikationssystem (TAS), Nukleinsäuresequenz-basierte Amplifikation (NASBA), Rolling Circle Amplifikation (RCA), Transkriptions-vermittelte Amplifikation (TMA), selbsterhaltende Sequenzreplikation (3SR), Qβ-Amplifikation und (thermostabile) Helikase-abhängige Amplifikation ((t) HAD).

4. Verfahren gemäß einem der Ansprüche 2 bis 3, wobei die Loci gleichzeitig amplifiziert werden.

5. Verfahren gemäß Anspruch 3 oder 4, wobei die Amplifikationsreaktion multiplex PCR ist.

6. Verfahren gemäß einen der Ansprüche 2 bis 5, zusätzlich umfassend den Schritt des Trennens der Amplifikationsprodukte nach ihrer Größe oder ihrem Molekulargewicht vor dem Nachweisen der Amplifikationsprodukte.

7. Verfahren gemäß Anspruch 2, wobei die Amplifikationsreaktion eine real-time multiplex Polymerasekettenreaktion ist, das Verfahren umfassend den Schritten:
(i) gleichzeitiges Amplifizieren von mindestens sechs Loci auf Nukleinsäuretemplaten unter Verwendung von Primerpaaren, die im Wesentlichen komplementär zu Sequenzen sind, die die Loci flankieren,
(ii) Nachweisen der Amplifikationsprodukte unter Verwendung von mindestens einer Oligonukleotidsonde für jeden zu amplifizierenden I'Locus, wobei die Oligonukleotidsonde im Wesentlichen komplementär zu einer Sequenz an dem zu amplifizierenden Locus ist,
(iii) wobei ein Fluoreszenzfarbstoff kovalent an jede Oligonukleotidsonde gebunden ist und wobei zur Differenzierung der mindestens sechs Sonden mindestens sechs verschiedene Farbstoffe verwendet werden, wobei die Farbstoffe ausgewählt sein können aus der Gruppe, umfassend:
i. ein erster Farbstoff, der 6-FAM oder 5-FAM, oder eine Mischung davon ist,
ii. ein zweiter Farbstoff, der DY-530, HEX oder ATTO 532 oder eine Mischung davon ist,
iii. ein dritter Farbstoff, der ATTO550 oder DY-555 oder eine Mischung davon ist,
iv. ein vierter Farbstoff der ATTO565, DY510-XL oder ROX oder eine Mischung davon ist,
v. ein fünfter Farbstoff der DY632 oder DY520-XL oder eine Mischung davon ist,
vi. und ein sechster Farbstoff der Chromeo 494 ist.

8. Verfahren gemäß einem der Ansprüche 1 bis 7, wobei
a. der erste Farbstoff 6-FAM ist,
b. der zweite Farbstoff ATTO550 ist,
c. der dritte Farbstoff ATTO565 ist,
d. und der vierte Farbstoff Chromeo 494 ist.

9. Verfahren gemäß einem der Ansprüche 1 bis 8, wobei mehr als sechs Farbstoffe verwendet werden und diese sin:
a. 6-FAM,
b. DY-530,
c. ATTO550,
d. ATTO565,
e. DY632 und,
f. Chromeo 494.

10. Verfahren gemäß einem der Ansprüche 2 bis 9, wobei mindestens ein Farbstoff an einem Oligonukleotidgrößenmarker kovalent gebunden ist.

11. Zusammensetzung umfassend:
(i) eine erste Nukleinsäure, an die ein Fluoreszenzfarbstoff, ausgewählt aus der Gruppe bestehend aus DY-530, HEX oder ATTO 532 oder eine Mischung davon, kovalent gebunden ist,
(ii) eine zweite Nukleinsäure, an die ein Fluoreszenzfarbstoff, ausgewählt aus der Gruppe bestehend aus DY-530, Hex oder ATTO 532 oder eine Mischung davon, kovalent gebunden ist,
(iii) eine dritte Nukleinsäure, an die ein Fluoreszenzfarbstoff, ausgewählt aus der Gruppe bestehend aus ATTO550 und DY-555, kovalent gebunden ist,
(iv) eine vierte Nukleinsäure, an die ein Fluoreszenzfarbstoff, ausgewählt aus der Gruppe bestehend aus ATTO565, DY510-XL und ROX, kovalent gebunden ist,
(v) eine fünfte Nukleinsäure, an die ein Fluoreszenzfarbstoff, ausgewählt aus der Gruppe bestehend aus DY632 oder DY520-XL oder eine Mischung davon, kovalent gebunden ist, und
(vi) eine sechste Nukleinsäure, an die ein Fluoreszenzfarbstoff gebunden ist, wobei der Farbstoff Chromeo 494 ist.

12. Kit für die Multiplex-PCR oder PCR, umfassend mindestens sechs verschiedene Fluoreszenzfarbstoffe, die zur kovalenten Bindung an Nukleotide, Primer, Größenmarker oder Oligonukleotidsonden, die in der Multiplex-PCR verwendet werden, geeignet sind, wobei die sechs Farbstoffe aus den folgenden sechs Gruppen ausgewählt sind:
i. 6-FAM oder 5-FAM oder eine Mischung davon,
ii. DY-530, HEX oder ATTO 532 oder eine Mischung davon,
iii. ATTO550 oder DY-555 oder eine Mischung davon,
iv. ATTO565, DY510-XL oder ROX oder eine Mischung davon,
v. DY632 oder DY520-XL oder eine Mischung davon, und
vi. Chromeo 494.

13. Kit gemäß Anspruch 12 umfassend mindestens sechs verschiedene Farbstoffe, wobei es sich um:
a. 6-FAM,
b. DY-530,
c. ATTO550,
d. ATTO565,
e. DY632 und,
f. Chromeo 494.

## Revendications

1. Procédé de détection simultanée d'au moins six acides nucléiques marqués avec un colorant lié de manière covalente dans un échantillon, comprenant les étapes suivantes:
Détecter l'émission de fluorescence des colorants fluorescents lors d'une excitation, dans lequel les au moins six colorants liés aux acides nucléiques sont choisis dans les groupes suivants:
i. 6-FAM ou 5-FAM ou un mélange de ceux-ci liés de manière covalente à un acide nucléique,
li DY-530, HEX ou ATTO 532 ou un mélange de ceux-ci liés manière covalence à un acide nucléique,
ii. ATTO550 ou DY-555, ou un mélange de ceux-ci, liés de manière covalente à un acide nucléique,
iv. ATTO565, DY510-XL ou ROX ou un mélange de ceux-ci liés de manière covalente à un acide nucléique,
v. DY632 ou DY520-XL ou un mélange de ceux-ci liés manière covalente à un acide nucléique,
vi. Chrome 494 lié à un acide nucléique.

2. Procédé de détection simultanée d'au moins six produits d'amplification à partir d'une réaction d'amplification comprenant les étapes suivantes:
(i) amplifier cinq locus ou plus sur des sondes d'acide nucléique en utilisant des amorces ou des paires d'amorces qui sont sensiblement complémentaires aux séquences bordant les loci; et
(ii) detecter des produits d'amplification par détection par fluorescence de produits d'amplification marqués par fluorescence, ou alternativement par détection par fluorescence de sondes oligonucléotidiques marquées par fluorescence qui sont complémentaires aux séquences situées au niveau des loci des produits d'amplification,
(iii) dans lequel un colorant fluorescent est lié de manière covalente au produit d'amplification et/ou à la sonde oligonucléotidique et/ou à l'amorce et/ou à au moins une amorce de la paire d'amorces et facultativement à un marqueur de taille,
(iv) et dans lequel au moins six colorants différents sont utilisés pour différencier les au moins six produits d'amplification, ces colorants pouvant être choisis dans le groupe comprenant:
i. un premier colorant qui est 6-FAM ou 5-FAM, ou un mélange de ceux-ci,
ii. un second colorant qui est DY-530, HEX ou ATTO 532 ou un mélange de ceux-ci,
iii. un troisième colorant qui est ATTO550 ou DY-555 ou un mélange de ceux-ci
iv. un quatrième colorant qui est le ATTO565, le DY510-XL ou le ROX ou un mélange de ceux-ci,
iv. un cinquième colorant qui est le DY632 ou le DY520-XL ou un mélange de ceux-ci,
v. et un sixième colorant qui est le Chromeo 494.

3. Procédé selon la revendication 2, dans lequel la réaction d'amplification est choisie dans le groupe comprenant la réaction de polymérase en chaîne (PCR), la réaction réaction de ligature en chaîne (LCR), le système d'amplification à base de transcription (TAS), l'amplification isothermique de séquences nucléiques (NASBA), l'amplification par cercle roulant (RCA), l'amplification par transcription (TMA), le systéme auto-entretenu de réplication de séquences (3SR), l'amplification Qβ et l'amplification (thermostable) dépendante de l'hélicase ((t) HAD).

4. Procédé selon l'une quelconque des revendications 2 à 3, dans lequel les locus sont amplifiés simultanément.

5. Procédé selon la revendication 3 ou 4, dans lequel la réaction d'amplification est une PCR multiplexe.

6. Procédé selon l'une quelconque des revendications 2 à 5, comprenant en outre l'étape consistant à séparer les produits d'amplification par taille ou poids moléculaire avant de détecter les produits d'amplification.

7. Procédé selon la revendication 2, dans lequel la réaction d'amplification est une réaction en chaîne par polymérase multiplexe en temps réel, le procédé comprenant les étapes consistant à:
(i) amplifier simultanément au moins six loci sur des matrices d'acide nucléique en utilisant des paires d'amorces qui sont sensiblement complémentaires aux séquences bordant les loci,
(ii) détecter les produits d'amplification en utilisant au moins une sonde oligonucléotidique pour chaque locus à amplifier, la sonde oligonucléotidique étant sensiblement complémentaire d'une séquence du locus à amplifier,
(iii) dans lequel un colorant fluorescent est lié de manière covalente à chaque sonde oligonucléotidique et dans lequel au moins six colorants différents sont utilisés pour différencier les au moins six sondes, ces colorants pouvant être choisis dans le groupe comprenant:
i. un premier colorant qui est 6-FAM ou 5-FAM, ou un mélange de ceux-ci,
ii. un second colorant qui est DY-530, HEX ou ATTO 532 ou un mélange de ceux-ci,
iii. un troisième colorant qui est ATTO550 ou DY-555 ou un mélange de ceux-ci
iv. un quatrième colorant est le ATTO565, le DY510-XL ou le ROX ou un mélange de ceux-ci,
vi. un cinquième colorant est le DY632 ou le DY520-XL ou un mélange de ceux-ci,
vii. et un sixième colorant est le Chromeo 494.

8. Procédé selon l'une des revendications 1 à 7, dans lequel
a. le premier colorant est le 6-FAM,
b. le deuxième colorant est ATTO550,
c. le troisième colorant est ATTO565,
d. et le quatrième colorant est Chromeo 494.

9. Procédé selon l'une quelconque des revendications 1 à 8, dans lequel plus de six colorants sont utilisés et ceux-ci sont:
a. 6-FAM,
b. DY-530
c. ATTO550,
d. ATTO565,
e. DY632 et
f. Chromeo 494.

10. Procédé selon l'une quelconque des revendications 2 à 9, dans lequel au moins un colorant est lié de manière covalente à un marqueur de taille d'un oligonucléotide.

11. Composition comprenant:
(i) un premier acide nucléique auquel est lié de manière covalente, un colorant fluorescent choisi dans le groupe constitué de DY-530, HEX ou ATTO 532 ou un mélange de ceux-ci,
(ii) un second acide nucléique auquel est lié de manière covalente, un colorant fluorescent choisi dans le groupe constitué de DY-530, hex ou ATTO 532 ou un mélange de ceux-ci,
(iii) un troisième acide nucléique auquel est lié de manière covalente, un colorant fluorescent choisi dans le groupe constitué par ATTO550 et DY-555,
(iv) un quatrième acide nucléique auquel est lié de manière covalente un colorant fluorescent choisi dans le groupe constitué par ATTO565, DY510-XL et ROX,
(v) un cinquième acide nucléique auquel est lié de manière covalente un colorant fluorescent choisi dans le groupe constitué de DY632 ou DY520-XL ou d'un mélange de ceux-ci, et
(vi) un sixième acide nucléique auquel est lié un colorant fluorescent, le colorant etant chromo 494.

12. Un kit pour PCR multiplexe ou PCR comprenant au moins six colorants fluorescents différents, favorables à une liaison covalente à des nucléotides, des amorces, des marqueurs de taille ou des sondes oligonucléotidiques utilisés dans la PCR multiplexe, les six colorants étant choisis parmi les six groupes suivants:
i. 6-FAM ou 5-FAM ou un mélange de ceux-ci,
ii. DY-530, HEX ou ATTO 532 ou un mélange de ceux-ci,
iii. ATTO550 ou DY-555 ou un mélange de ceux-ci,
iv. ATTO565, DY510-XL ou ROX ou un mélange de ceux-ci,
v. DY632 ou DY520-XL ou un mélange de ceux-ci, et
vi. Chromeo 494.

13. Le kit selon la revendication 12 comprenant au moins six colorants différents, qui sont:
a. 6-FAM,
b. DY-530
c. ATTO550,
d. ATTO565,
e. DY632 et
f. Chromeo 494.
